# EUROPEAN PATENT APPLICATION

(11) **EP 4 740 972 A1**
(43) Date of publication of application: **13.05.2026**
(21) Application number: 24383215.1
(22) Date of filing: 07.11.2024
(51) Int. Cl.: A61L 27/46, B33Y 70/00, B33Y 80/00

(54) **METHOD FOR PRODUCING A 3D PRINTED BONE GRAFT AND 3D PRINTED BONE GRAFT**

(71) Applicant: Universitat Politècnica De Catalunya, 08034 Barcelona (ES)
(72) Inventor: GINEBRA MOLINS, Maria Pau, 08019 Barcelona (ES); FAGOTTO CLAVIJO, Roberto, 08019 Barcelona (ES); LODOSO TORRECILLA, Irene, 08030 Barcelona (ES); DÍEZ ESCUDERO, Anna, 08019 Barcelona (ES)
(74) Representative: Torner, Juncosa I Associats, SL

(57) **Abstract**

A method for producing a 3D printed bone graft and a 3D printed bone graft are proposed. The method comprises a) preparing a photocurable composite resin composition by mixing a photocurable biocompatible polymeric resin, comprising one or more monomers/oligomers and a photoinitiator, with a hydrolysable calcium phosphate-based ceramic material; b) manufacturing a 3D printed bone graft using the photocurable resin composition through 3D printing with a light-based 3D printing system; c) cleaning and removing uncured resin or particles from the 3D printed bone graft by ultrasonic rinsing in a solvent; d) providing consolidation to the 3D printed bone graft by: d1) sublimating liquid contained in the resin by freeze-drying the manufactured 3D printed bone graft; and/or d2) hydrolyzing the hydrolysable calcium phosphate-based ceramic material to calcium deficient hydroxyapatite (CDHA).

## Description

### TECHNICAL FIELD

The present invention relates to 3D printing compositions comprising ceramic and polymeric materials and to methods for printing 3D ceramic and polymeric objects. Specifically, the present invention relates to a method for producing a 3D printed bone graft and a 3D printed bone graft manufactured using the method.

### BACKGROUND OF THE INVENTION

Bone defects caused by trauma, osteoporotic fractures, infection and tumor resection pose a great clinical and socio-economic problem. The field of bone grafting is gradually shifting from autologous or allogeneic grafts to synthetic substitutes. Among them, calcium phosphate-based materials, particularly hydroxyapatite, are extensively utilized due to their biocompatibility and osteogenic potential.

Bone grafts should exhibit excellent osteoconductivity, hence the ability to lead a rapid colonization by cells, which adhere, migrate, grow, and divide. Consequently, interconnected macroporosity (>100 µm) is highly desired to promote osteoconduction. These macroporous grafts are commonly referred to as scaffolds, which are a temporary mechanical structure designed to create an optimal environment for bone remodeling with minimal complications. These structures are widely employed in bone repair due to their ability to fill the defective area, provide mechanical support, and guide the growth of new tissue. Scaffolds ought to facilitate the resorption of the synthetic material, and its replacement with newly formed natural bone. Additionally, it is highly desired for scaffolds to actively influence the formation of new bone, accelerating the healing process, which is known as osteoinductivity, being the ability to favor the differentiation of mesenchymal stem cells into bone cells.

With the advent of 3D-printing technologies, the field of bone grafting has seen remarkable advancements. 3D printing has enabled the development of highly personalized and high-performing bone grafts. Extrusion-based techniques such as Direct Ink Writing (DIW) or Fused Filament Fabrication (FFF) are widely used for printing high-loaded ceramic bone grafts. Extrusion-based techniques involve the layer-by-layer extrusion of an ink through a nozzle to build an implant. Despite the significant progress in this area, certain limitations still hinder the widespread application of extrusion-based technology, such as precision in structural integrity and scalability.

To address these limitations, light-based technologies have been increasingly applied. Light-based printing techniques use various optical arrays and devices to direct light precisely onto specific areas containing photocurable resins, which are then photocrosslinked. These techniques encompass a wide range of systems, including Digital Light Processing (DLP) with Digital Micromirror Devices (DMD-DLP) or Liquid Crystal Displays (LCD-DLP), Stereolithography (SLA), masked SLA (mSLA), Continuous Liquid Interface Production (CLIP), Two-Photon Polymerization (2PP), and Volumetric 3D Printing (V3DP). Each of these methods employs light to cure resin, with higher resolution compared to extrusion-based techniques, enabling the creation of highly detailed structures..

In this context, patent US10835390-B2 introduces a method for manufacturing a bone graft material using 3D printing of a DLP system. The method includes dispersing a powder material including calcium phosphate-based ceramics in a solvent; recovering the calcium phosphate-based ceramics by removing the solvent from a solution in which the calcium phosphate-based ceramic material is dispersed; producing a photocurable resin composition by adding a binder resin including a crosslinking agent and a photoinitiator to the calcium phosphate-based ceramics; performing 3D rapid prototyping on a bone graft material molded body from the composition for a bone graft material by 3D printing of a DLP system; and debinding and sintering the materials remaining in the bone graft material molded body subjected to prototyping. In this method, unlike the present invention, to induce growth of the particles and improve strength, the materials have to be subjected to additional debinding and sintering steps.

Other methods utilizing DLP to manufacture 3D-printed objects for bone defect regeneration and repair are known by scientific articles [1-3].

In view of the foregoing, it is an object of the present invention to enhance the disadvantages of the prior art, or to provide a useful alternative.

### References:

[1] DLP printing of hydrogel/calcium phosphate composites for the treatment of bone defects. I.I. Preobrazhenskiy et al. Open Ceramics. Volume 6, June 2021.
[2] Hydroxyapatite-Resin Composites Produced by Vat Photopolymerization and Post-Processing via In Situ Hydrolysis of Alpha Tricalcium Phosphate. Carolina Oliver-Urrutia et al. Ceramics 2023, 6(4), 2282-2294.
[3] Customized bioceramic scaffolds and metal meshes for challenging large-size mandibular bone defect regeneration and repair. Bin Zhang, et al. Regenerative Biomaterials, Volume 10, 2023.

### DESCRIPTION OF THE INVENTION

It is an object of present invention to provide a method for producing a 3D structure, particularly a 3D printed bone graft, for bone tissue engineering applications.

To that end, according to a first aspect, a method for producing a 3D printed bone graft, for example, a bone regeneration product such as a scaffold, is provided. The method comprises a) preparing a photocurable composite resin composition by mixing a photocurable biocompatible polymeric resin, comprising one or more monomers/oligomers and a photoinitiator, with a hydrolysable calcium phosphate-based ceramic material; b) manufacturing a 3D printed bone graft using the photocurable composite resin composition through 3D printing with a light-based 3D printing system; c) cleaning and removing uncured resin from the 3D printed bone graft by ultrasonic rinsing in an appropriate solvent; d) providing consolidation to the 3D printed bone graft by at least one of sublimating liquid contained in the resin by freeze-drying the manufactured 3D printed bone graft or hydrolyzing the hydrolysable calcium phosphate-based ceramic material to calcium deficient hydroxyapatite (CDHA).

The light-based 3D printing system may comprise light-projection devices or lasers exposures such as Digital Light Processing (DLP) with Digital Micromirror Devices (DMD-DLP) or Liquid Crystal Displays (LCD-DLP), Stereolithography (SLA), masked SLA (mSLA), Continuous Liquid Interface Production (CLIP), Two-Photon Polymerization (2PP), Volumetric 3D Printing (V3DP), or any other light projection-based technology capable of curing the resin composition. In some particular embodiments, it comprises a LCD-DLP system.

In some embodiments, step b) comprises irradiating the photocurable resin composition using a light source having a light wavelength of 280 nm to 460 nm.

In some embodiments, step a) further comprises using a photoabsorber in the wavelength range between 280 nm to 460 nm.

In some embodiments, step a) further comprises using a dispersant in the range between 0.5 and 5 wt% relative to the hydrolysable calcium phosphate-based ceramic material.

In some embodiments, the hydrolyzation step is carried out by immersing the 3D printed bone graft in distilled water at a given temperature and pressure. For example, the given temperature can be comprised in the range between 20°C and 150°C and the pressure in the range between 0.5 and 2 atm. In some embodiments, the 3D printed bone graft is immersed for a period of time comprised between 10 minutes and 15 days.

In some embodiments, the hydrolysable calcium phosphate-based ceramic material comprises α-tricalcium phosphate (α-TCP). In some embodiments, it comprises β-tricalcium phosphate (β-TCP). In some embodiments, it comprises a combination of α-TCP and β-TCP. In other embodiments, it can comprise other calcium phosphates, such as tetracalcium phosphates (TTCP), dicalcium phosphate dihydrate and anhydrous (DCPD, DCPA) among others, and/or ceramic particles (e.g. hydroxyapatite (HA), more specifically calcium deficient hydroxyapatite).

In some embodiments, the one or more monomers/oligomers comprise one or more acrylated monomers/oligomers such as poly (ethylene glycol) diacrylate (PEGDA); Poly(ethylene glycol) dimethacrylate PEGMA; methacrylated gelatin GelMA in a concentration ranging between 5 and 90 wt%.

In some embodiments, the photoinitiator comprises a single material or a mixture of two or more materials selected from: Lithium phenyl-2,4,6-trimethylbenzoylphosphinate, diphenyl(2,4,6-trimethylbenzoyl)phosphine oxide/2-hydroxy-2-methylpropiophenone, (benzene)tricarbonylchromium,4,4'-bis(diethylamino)benzophenone, phenanthrenequinone, bis(2,4,6-trimethylbenzoyl)-phenylphosphineoxide,2,4,6 trimethylbenzoyl-diphenyl-phosphineoxide, 2-benzyl-2-dimethylamino-1-(4-orpholinophenyl)-butanone-1,bis(eta5-2,4-cyclopentadien-1-yl)-bis(2,5-difluoro-3-(1H-yrrol-1-yl)-henyl)titanium, and diphenyl(2,4,6-trimethylbenzoyl)phosphine oxide, tris(2,20-bipyridyl)ruthenium (II) chloride hexahydrate (Ru) / sodium persulfate (SPS).

In some embodiments, the photoinitiator is comprised in an amount between 0.25 to 2 wt.% with respect to the total weight of the photocurable resin.

In some embodiments, the hydrolysable calcium phosphate-based ceramic material is comprised in the range between 30 and 70 wt. % with respect to the total weight of the photocurable composite resin composition.

Another aspect of the present invention provides a 3D printed bone graft produced by the method of the first aspect.

In some embodiments, the 3D printed bone graft comprises a ceramic matrix and a polymeric matrix, wherein a) the ceramic matrix comprises an interlocked crystalline phase including calcium deficient hydroxyapatite, and might comprise α-TCP and/or β-TCP, in a range of 30 to 70% wt. with respect to the total weight of the composite resin.

In some embodiments, the 3D printed bone graft comprises a bone regeneration product with the CDHA phase at a percentage of 5 to 96% of the ceramic phase and/or the ceramic comprises α-TCP and/or β-TCP.

In some embodiments, the bone regeneration product can comprise one or more of the following features:
- a polymeric matrix made from one or more acrylated monomers/oligomers, particularly PEGDA, GeIMA, or a combination thereof;
- a specific surface area (SSA) in the range of 1 to 30 m²/g, as determined by nitrogen adsorption and BET analysis;
- crystalline phases from the ceramic part comprising α-TCP, β-TCP and HA;
- the macroporosity is comprised in the range between 10 to 80%, and the nano/microporosity in the range between 10 to 30%.

### BRIEF DESCRIPTION OF THE DRAWINGS

The previous and other advantages and features will be more fully understood from the following detailed description of embodiments, with reference to the attached figures, which must be considered in an illustrative and non-limiting manner, in which:

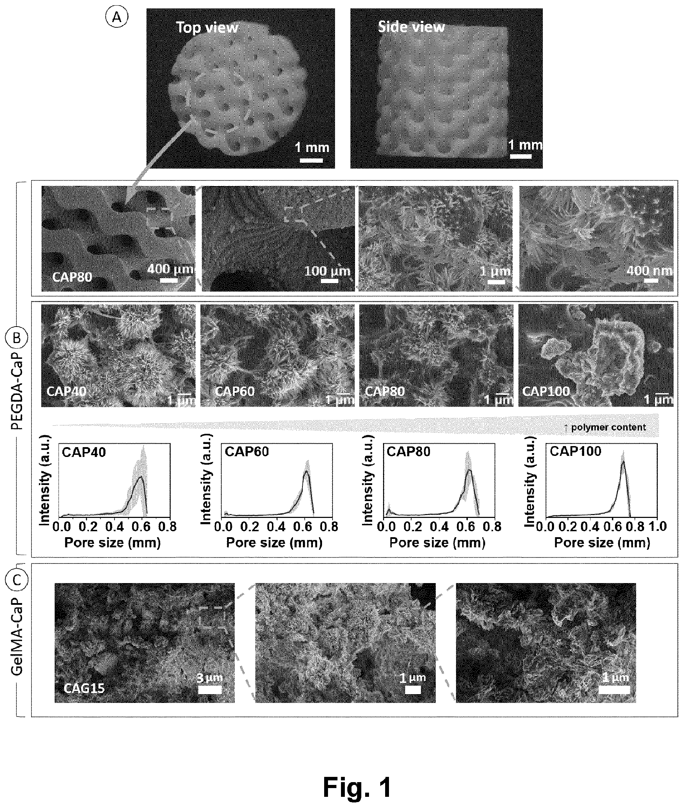

Fig. 1 illustrates some results of different 3D bone graft obtained by the proposed method using PEGDA and GeIMA. (A) Top and side views of CAP80 3D-printed bone graft. (B) Micrographs of PEGDA-based composite resin: CAP80 scaffold at various magnifications, along with CAP40, CAP60, and CAP100, illustrating CDHA needle formation after water immersion. All compositions yielded scaffolds with pore size around 600 µm. (C) Micrographs of GelMA-based composite resin: CAG15 showing CDHA needle formation post-immersion in water. The composition of CAP40, CAP60, CAP80, and CAP100, and CAG15 are displayed in table 1 and table 2.

### DETAILED DESCRIPTION OF THE INVENTION AND OF PREFERRED EMBODIMENTS

The present invention leverages light-based 3D printing for the fabrication of personalized bone grafts. This allows printing structures that mimic trabecular bone with highly interconnected spaces, which is not possible with extrusion-based techniques such as Direct Ink Writing (DIW).

Additionally, the innovative strategy of the present invention lies in the provision of a method for producing a 3D printed bone graft using a photocurable polymeric resin combined with a hydrolysable calcium phosphate-based ceramic material. Specifically, the invention combines a photocurable polymeric resin, photoinitiator, and a hydrolysable ceramic component. This composite resin composition is then used in a light-based 3D printing system to manufacture the 3D bone graft, with the curing process initiated by light within a wavelength range of 280-460 nm.

Once cleaned, the 3D printed bone graft can be consolidated using different strategies. One of the strategies involves freeze-drying to sublimate the liquid content in the resin, while the second strategy focuses on hydrolyzing the calcium phosphate material to form calcium-deficient hydroxyapatite (CDHA). In some examples, this hydrolysis is achieved by immersing the graft in distilled water at temperatures ranging from 20°C to 150°C and pressures between 0.5 to 2 atmospheres for periods ranging from 10 minutes to 15 days. Additional additives, such as photoabsorbers and/or dispersants, can be incorporated to optimize the printing process and material characteristics. The hydrolysis to CDHA will take place when the 3D printed bone graft enters in contact with water or an aqueous solution, or with the body fluids once it is implanted. The hydrolysis process results in a microstructural change, with the consequent enhancement of the mechanical properties.

The final synthetic bone graft, which may include a scaffold structure for bone regeneration, is suitable for implantation into animal bodies. The method ensures a stable, biocompatible product that can be tailored for specific bone regeneration applications through the careful selection of materials and processing conditions.

The photocurable polymeric resin can be prepared by dissolving the monomers/oligomers in water or any other suitable solvent until a homogeneous mixture is achieved. An absorber can then be added, if necessary, depending on target resolution, followed by thorough mixing to ensure uniformity. Next, the photoinitiator is introduced and homogenized, followed by the addition of a dispersant, which is similarly mixed until being fully homogenized. Finally, the hydrolysable calcium phosphate-based ceramic material is added and mixed by centrifugal mixer. The 3D printing process involves configuring the designed models (that may include models from medical images and their conversion to printer adaptable language, i.e. gcode), preparing the printer, adjusting the printing settings (such as light exposure time and intensity), and executing the printing itself. Once printed, the bone grafts are rinsed with water and subjected to ultrasound treatment to remove any uncured or unpolymerized monomers/oligomers and additives.

For consolidation, the printed bone grafts can be freeze-dried by sublimating the solvent, thereby preserving the hydrolysable potential of the ceramic phase. The hardening of the ceramic phase occurs through hydrolysis of the calcium phosphate, while the polymeric phase remains intact. This hardening process can be performed directly during implantation into animal bodies or by pre-hardening the bone grafts before implantation by placing them in contact with water. The physicochemical properties of the bone grafts vary depending on the format-whether they are pre-hardened (hardened after printing, prior to implantation) or unset (designed to harden upon implantation).

In the following, some particular examples of the materials and preparation methods used/proposed by the present invention are detailed.

### Ceramic powder synthesis

In a particular embodiment, the powder phase comprising α-TCP and β-TCP was synthesized from calcium carbonate (CaCO₃) and dicalcium phosphate (CaHPOt) reactants by solid-state reaction. Briefly, reactants were homogenously mixed adjusting the Ca/P molar ratio to 1.5, and were thermally treated to allow the decomposition into calcium pyrophosphate (Ca₂P₂O₇) and calcium oxide (CaO), which are the precursors of β-TCP, obtained through solid-state reaction at 1100°C. β-TCP was obtained by maintaining at 1100°C for 9 hours, and left slowly cooling in the furnace. On the other hand, α-TCP synthesis was performed at 1400 °C for 2 hours (above the transus-temperature, of about 1125 °C), and was retained at room temperature by air quenching, leaving no time for α-TCP to transform to β-TCP. The material block was gathered and milled several times at 500 rpm intermittently to avoid excessive heating, using an agata jar filled with big and small agata balls (20 mm, and 5 mm diameter respectively). Fine powder with dimensions under 40 µm resulted after overnight sieving. The synthesized fine powders were mixed with resins at a ratio of 1:1.

### Preparation of photocurable polymeric resin

In a particular embodiment, the prepolymer solution contained Poly (ethylene glycol) diacrylate (PEGDA, Mw=700 g/mol) as crosslinkable monomer/oligomer and Lithium phenyl-2,4,6-trimethylbenzoylphosphinate (LAP) as photoinitiator. Tartrazine 89% pure and Orange II sodium salt were used as photoabsorber molecules. The selected dispersant was Disperbyk 111. The self-developed build plate, consisting of a microscope slide, was treated using 3-(Trimethoxysilyl)propyl methacrylate, 98% pure, used as binding silane.

In a particular embodiment, the proposed photocurable resin composition comprises crosslinkable monomers/oligomers with acrylate terminating groups, a photoinitiator that starts the polymerization reaction of the monomers/oligomers by free radical polymerization, and a dye that guarantees printing resolution preventing the crosslinking at non-desired areas due to scattered light.

In a particular embodiment, different formulations based on PEGDA are proposed, varying PEGDA (Mw=700 g/mol) prepolymer content at 40-60-80-100 wt.% regarding the water content. LAP, and tartrazine/Orange G (photoinitiator and photoabsorber molecules respectively) were added as weight percentages regarding PEGDA and water mixture (polymeric resin). The corresponding formulations are listed in Table 1.

**Table 1: formulation of PEGDA photocurable polymeric resins. The used dyes were Tartrazine (Tz) and Orange G (Og), and their values/concentrations are expressed with respect to the total polymeric resin.**

| *Photocurable PEGDA monomer*/*oligomer-based resins* | | | | |
|---|---|---|---|---|
| *Nomenclature* | PEGDA (wt.%) | Distilled H₂O (wt.%) | LAP wt.% | ( Dye wt.% |
| *R40* | 40 | 60 | 0.75 | 0.19 (Tz) |
| *R60* | 60 | 40 | 0.75 | 0.19 (Tz) |
| *R80* | 80 | 20 | 0.75 | 0.19 (Tz) |
| *R100* | 100 | 0 | 0.50 | 0.04 (Og) |

To start off, *R40, R60, and R80* resins were prepared by first adding PEGDA (Mw=700 g/mol) monomers/oligomers, LAP, and Tartrazine to distilled water, as seen in Table 1. The resins were then mixed in a dual asymmetric centrifugal mixer for five minutes at 2500 rpm until complete dissolution. On the other hand, *R100* resin, lacking water in its formulation, was prepared using Orange-G as photo-absorber instead of Tartrazine. The absence of water in R100 formulation makes the dissolution of tartrazine not possible, hence the use of Orange-G. The *R100* resin was first heavily sonicated for several minutes and then magnetically stirred at 1000 rpm overnight until complete dissolution of the components.

In another particular embodiment, the prepolymer solution contained methacrylated gelatine (GeIMA,) as crosslinkable monomer/oligomer and tris(2,20-bipyridyl)ruthenium (II) chloride hexahydrate (Ru)/ sodium persulfate (SPS) or Lithium phenyl-2,4,6-trimethylbenzoylphosphinate (LAP) as photoinitiators. Tartrazine was used as photoabsorber molecule. The selected dispersant was Disperbyk 111. The self-developed build plate, consisting of a microscope slide, was treated using 3-(Trimethoxysilyl)propyl methacrylate, 98% pure, used as binding silane.

To start off, *G10,* G15 resins were prepared by first adding GelMA monomers/oligomers, LAP or Ru/SPS and Tartrazine to distilled water. The resins were then mixed with a magnetic stirrer at 800 rpm until complete dissolution.

In a particular embodiment, different formulations based on methacrylated gelatine (GeIMA) are proposed, varying the GelMA content at 10-20 wt. % regarding the water content. Ru/SPS or LAP, and tartrazine (photoinitiators and photoabsorber molecules respectively) were added as weight percentages regarding GelMA and water mixture (polymeric resin).

**Table 2: formulation of GelMA photocurable polymeric resins. The used dye is Tartrazine (Tz) and its values/concentrations are expressed with respect to the total polymeric resin.**

| *Photocurable GelMA monomer*/*oligomer-based resins* | | | | | |
|---|---|---|---|---|---|
| *Nomenclature* | GelMA (wt.%) | Distilled H₂O (wt.%) | LAP wt.% | Ru/SPS mM/mM | Dye wt.% |
| *G10* | 10 | 90 | -- | 0.2/2 | 0.01 (Tz) |
| G15 | 15 | 85 | 0.75 | -- | 0.01 (Tz) |

### Preparation of composite photocurable resin with hydrolysable calcium phosphate-based ceramic material

Afterwards, Disperbyk 111 (2 wt.% of the total ceramic content) was added to each resin and mixed in a dual asymmetric centrifugal mixer at 2500 rpm for three minutes. Finally, the ceramic content was added (establishing the ceramic: resin ratio = 1:1), and was then mixed at 3500 rpm for five minutes, resulting in homogeneous and low viscous resin suitable for LCD-DLP printing, i.e. RA40, RA60, RA80, and RA100, meaning resin + αTCP, and the percentage of PEGDA in the resin (see Table 2). The photocurable composite resins, consisting of PEGDA and/or GelMA and α-TCP or β-TCP, were named as C followed by A or B (alpha or beta-TCP respectively) + the resin's prepolymer nature (P or G for PEGDA or Gelatin respectively) and concentration (10, 40, 60, 80, 100). For instance, CAP80 stands for composite resin consisting of alpha-TCP and a polymeric resin containing 80 wt.% PEGDA prepolymers.

**Table 3: Formulation of photocurable composite resins**

| *Polymeric Resin* | *Ceramic filler* | *Composite resin (Polymeric resin: Ceramic 1:1)* |
|---|---|---|
| *R40* | α-TCP | CAP40 |
| *R60* | α-TCP | CAP60 |
| *R80* | α-TCP | CAP80 |
| *R100* | α-TCP | CAP100 |
| *R80* | β-TCP | CBP80 |
| *G10* | α-TCP | CAG10 |

### Bone graft 3D printing

In a particular embodiment, bone grafts were manufactured using a LCD-DLP printer composed of an LCD screen working at 405 nm wavelength. Cylindrical bone grafts (6 mm diameter x 6 mm height), and disks (5 mm diameter x 1 mm height), were designed with gyroid triply periodic minimal surface (TPMS), modeled by the open-source *Scaffolder* software. The targeted porosity and pore size were 50% and 600 µm respectively. The program works as an STL mediator, which receives a raw design as input, applies a mathematical algorithm to the design's structure, and gives back the design modified with the programed TPMS topology. This program was developed to create a three-dimensional architectural framework with a targeted pore size and porosity. The selected topology was chosen to enhance the scaffold's mechanical properties by increasing elasticity, sufficient flexural resistance, and desirable stress distribution and to promoted cell proliferation when compared to common grid-like scaffolds. Plus, gyroid is vastly used throughout literature, and outperforms in surface to volume ratio with 4.1 compared to 2.3, and 2.8 from Schwarz and Diamond TPMS. The .stl files were imported in a 3D slicing software. The layer thickness was set to 50 µm, and the exposure times are shown in Table 3.

After printing, bone grafts were thoroughly cleaned to remove uncured resin inside the structure, by ultrasonically rising them in distilled water (as PEGDA and GelMA monomers/oligomers are water soluble). After printing, the scaffolds were immersed in distilled water at 37°C to allow hardening reaction of α-TCP to calcium deficient hydroxyapatite (CDHA).

**Table 4. Light exposure times for each specific resin formulation.**

| *Composite resin* | *Bottom exposure (s)* | *Layer exposure (s)* |
|---|---|---|
| *CAP40* | 150 | 100 |
| *CAP60* | 100 | 40 |
| *CAP80* | 100 | 40 |
| *CAP100* | 100 | 30 |
| *CBP80* | 100 | 40 |
| *CAG10* | 40 | 8 |

Note that these exposure parameters are specific for the printing device used, and they will require further adjustments if the formulations vary (as is the case of using other monomers) or the printing machine varies.

Fig. 1 shows optical images of two manufactured 3D printed scaffolds consisting of CAP80 3D printed bone grafts. It is clearly seen that the formulated resins are suitable to create complex gyroid-structured scaffolds, with high resolution and details. The details of pore characteristics, and pore size distribution of samples can be quantified by X-ray micro-computed tomography (µ-CT), among others, for the quantification of the macroporosity and pore size distribution. In this particular example, the analyses were carried with samples immersed in water, to avoid water leaving the hydrogel which translated into a shrinking in dimensions which would give unreliable structural data. Scaffolds were printed with an interconnected macroporous structure, with open-macroporosity percentage values of CAP40: 51.07 ± 2.86 %; CAP60: 51.55 ± 0.81 %; CAP80: 52.66 ± 1.99 %; and CAP100: 46.67 ± 4.67 %; which correlate fairly well the designed 50% open-macroporosity. These results suggest the accurate customization that the proposed formulated ceramic-loaded resins present. Moreover, the scaffolds showed a pore size distribution ranging the 600 µm, illustrated in Fig. 1, wide enough for cells and vessels colonization.

The morphology of the scaffolds obtained with PEGDA (CAP40, CAP60, CAP80 and CAP100) and GelMA (CAG10) was analyzed with an electron microscope at an accelerating voltage of 2 KV to 5KV and a beam current of 50-pA. Samples were previously coated with 10 nm C sputtering using a sputtering system. The obtained micrographs are shown in Fig. 1. The first-row zooms into the scaffold's surface, locating the need-like shaped crystals of CDHA embedded in a polymeric mantle-like matrix. This nanotopography, typical of the setting reaction of α-TCP cements are in accordance with the compositional characterization findings. In fact, neither PEGDA nor GelMA hindered the α-TCP hydrolysis to CDHA in any of the formulations. Furthermore, needle crystals were obtained in all scaffolds. This morphology is desirable because, in addition to being associated with osteogenic and osteoinductive effects, the presence of high aspect ratio nanotopographies may confer mechanobactericidal properties to the material. Moreover, variations on the aspect ratio of the needles were observed, being the CAP100 scaffolds, the ones showing shorter needles. This fact may be due to the lack of water in the hydrogel which, when immersing it into the water uptakes the surrounding water de-balancing the competition between the ceramic and the polymeric phases. This affects the crystal growth ratio of the ceramic phase, yielding a variety of aspect ratio of their crystals. More in detail, CAP40 exhibited huge and long aspect-ratio needles, which were diminished when increasing the polymeric content. In addition, this increase led to a larger partial-coverage of the needles in PEGDA.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

The embodiments and illustrations included herein show, by way of illustration and not of limitation, specific embodiments in which the subject matter may be practiced. Other embodiments may be utilized and derived there from, such that structural and logical substitutions and changes may be made without departing from the scope of this disclosure. Such embodiments of the inventive subject matter may be referred to herein individually or collectively bv the term "invention" merely for convenience and without intending to voluntarilv limit the scope of this application to any single invention or inventive concept, if more than one is, in fact, disclosed. Combinations of the above embodiments, and other embodiments not specifically described herein, will be apparent to those of skill in the art upon reviewing the above description.

The scope of the present invention is defined in the following set of claims.

## Claims

1. A method for producing a 3D printed bone graft, the method comprising the following steps:
a) preparing a photocurable composite resin composition by mixing a photocurable biocompatible polymeric resin, comprising one or more monomers/oligomers and a photoinitiator, with a hydrolysable calcium phosphate-based ceramic material;
b) manufacturing a 3D printed bone graft using the photocurable resin composition through 3D printing with a light-based 3D printing system;
c) cleaning and removing uncured resin or particles from the 3D printed bone graft by ultrasonic rinsing in a solvent;
d) providing consolidation to the 3D printed bone graft by:
d1) sublimating liquid contained in the resin by freeze-drying the manufactured 3D printed bone graft; and/or
d2) hydrolyzing the hydrolysable calcium phosphate-based ceramic material to calcium deficient hydroxyapatite, CDHA.

2. The method of claim 1, wherein step b) comprises irradiating the photocurable composite resin composition using a light source having a light wavelength of 280 nm to 460 nm.

3. The method of claim 1, wherein step a) further comprises using a photoabsorber in a wavelength range between 280 nm to 460 nm.

4. The method of claim 1, wherein step a) further comprises using a dispersant in a range between 0.5 and 5 wt% relative to the hydrolysable calcium phosphate-based ceramic material.

5. The method of any one of the previous claims, wherein step d2) comprises immersing the 3D printed bone graft in distilled water or an aqueous solution at a given temperature and pressure.

6. The method of claim 5, wherein the given temperature is comprised in the range between 20°C and 150°C and the pressure is comprised in the range between 0.5 and 2 atm.

7. The method of claim 5 or 6, wherein the 3D printed bone graft is immersed for a period of time comprised between 10 minutes and 15 days.

8. The method of any one of the previous claims, wherein the hydrolysable calcium phosphate-based ceramic material comprises α-tricalcium phosphate, β-tricalcium phosphate, or a mixture thereof.

9. The method of any one of the previous claims, wherein the one or more monomers/oligomers comprise an acrylated monomer/oligomer including poly (ethylene glycol) diacrylate, PEGDA; Poly(ethylene glycol) dimethacrylate, PEGMA; methacrylated gelatin GelMA monomer; and/or a combination thereof, in a concentration ranging between 5 and 90 wt%.

10. The method of any one of the previous claims, wherein the photoinitiator comprises a single material or a mixture of two or more materials selected from: Lithium phenyl-2,4,6-trimethylbenzoylphosphinate, diphenyl(2,4,6-trimethylbenzoyl)phosphine oxide/2-hydroxy-2-methylpropiophenone, (benzene)tricarbonylchromium,4,4'-bis(diethylamino)benzophenone, phenanthrenequinone, bis(2,4,6-trimethylbenzoyl)-phenylphosphineoxide,2,4,6 trimethylbenzoyl-diphenyl-phosphineoxide, 2-benzyl-2-dimethylamino-1-(4-orpholinophenyl)-butanone-1,bis(eta5-2,4-cyclopentadien-1-yl)-bis(2,5-difluoro-3-(1H-yrrol-1-yl)-henyl)titanium, and diphenyl(2,4,6-trimethylbenzoyl)phosphine oxide, tris(2,20-bipyridyl)ruthenium (II) chloride hexahydrate (Ru) / sodium persulfate (SPS).

11. The method of any one of the previous claims, wherein the photoinitiator is comprised in an amount between 0.25 to 2 wt.% with respect to the total weight of the photocurable polymeric resin.

12. The method of any one of the previous claims, wherein the hydrolysable calcium phosphate-based ceramic material is comprised in a range between 30 and 70 wt. % with respect to the total weight of the photocurable composite resin composition.

13. The method of any one of the previous claims, wherein the 3D printed bone graft comprises a bone regeneration product including a scaffold.

14. A 3D printed bone graft produced by a method for producing a bone graft, the method comprising the steps of:
a) preparing a photocurable composite resin composition by mixing a photocurable biocompatible polymeric resin, comprising one or more monomers and a photoinitiator, with a hydrolysable calcium phosphate-based ceramic material;
b) manufacturing a 3D printed bone graft using the photocurable resin composition through 3D printing with a light-based 3D printing system;
c) cleaning and removing uncured resin or particles from the 3D printed bone graft by ultrasonic rinsing in a solvent;
d) providing consolidation to the 3D printed bone graft by:
d1) sublimating liquid contained in the resin by freeze-drying the manufactured 3D printed bone graft; and/or
d2) hydrolyzing the hydrolysable calcium phosphate-based ceramic material to calcium deficient hydroxyapatite, CDHA.

15. The bone graft of claim 14, comprising a bone regeneration product including a scaffold.
